Europäisches Patentamt

European Patent Office

Office européen des brevets

(11) Veröffentlichungsnummer: **0 252 277**
**A2**

(12) **EUROPÄISCHE PATENTANMELDUNG**

(21) Anmeldenummer: 87107880.4

(22) Anmeldetag: 01.06.87

(51) Int. Cl.4: **C11D 1/83** , A61K 7/16

(30) Priorität: 09.06.86 DE 3619358

(43) Veröffentlichungstag der Anmeldung:
13.01.88 Patentblatt 88/02

(84) Benannte Vertragsstaaten:
AT BE CH DE FR GB IT LI NL SE

(71) Anmelder: Henkel Kommanditgesellschaft auf Aktien
Postfach 1100 Henkelstrasse 67
D-4000 Düsseldorf-Holthausen(DE)

(72) Erfinder: Breitzke, Willi
Am Falder 22
D-4000 Düsseldorf 13(DE)
Erfinder: Hensen, Hermann, Dr
Tizianweg 4
D-4010 Hilden(DE)

(54) **Wässrige Zubereitungen von Natriumlauryl-und-myristylsulfat mit niedrigem Trübungspunkt.**

(57) Wässrige klarflüssige Zubereitungen von Natriumlauryl-und myristylsulfat, mit einem Gehalt von

5 - 20 Gew.-% Natrium-lauryl-und/oder myristylsulfat

10 - 30 Gew.-% einer nichtionogenen oberflächenaktiven Ethylenoxidanlagerungsverbindung mit einem HBL-Wert von 12 - 18

10 - 20 Gew.-% eines Polyols mit 3 - 6 C-Atomen und 2 - 6 Hydroxylgruppen oder eines Polyethylenglycols

30 - 75 Gew.-% Wasser

zeichnen sich durch niedrige Trübungspunkte aus und eignen sich als Tensidkomponente bei der Herstellung von Zahnpasten.

EP 0 252 277 A2

## Wässrige Zubereitungen von Natriumlauryl-und -myristylsulfat mit niedrigem Trübungspunkt

Gegenstand der Erfindung sind wässrige, klarflüssige Zubereitungen von Natriumsalzen von linearen Alkylsulfaten, die sich aufgrund ihrer Zusammensetzung und anwendungstechnischen Eigenschaften besonders gut als Tensidkomponenten bei der Herstellung von Zahnpasten eignen.

Verdünnte wässrige Lösungen von Natrium-und -alkylsulfaten mit 12 - 16 C-Atomen neigen schon bei Temperaturen oberhalb +10°C zur Trübung und Abscheidung von ungelöstem Alkylsulfat. Der Grund dafür ist die verhältnismäßig geringe Wasserlöslichkeit dieser Alkylsulfate bei Temperaturen unterhalb der kritischen Lösungstemperatur bzw. der Krafft-Temperatur, d.h. der Temperatur oberhalb welcher eine verstärkte Mizellbildung die Wasserlöslichkeit sprunghaft erhöht. Diese Temperatur liegt z.B. für Natriumdodecylsulfat bei 8°C und für Natriumtetradecylsulfat bereits bei 20,5 °C (vergl. H. Lange und M. J. Schwuger, Kolloid-Zeitschrift und Zeitschrift für Polymere, Band 223 (1967), Seite 145 - 149).

Natrium-n-Alkylsulfate eigenen sich wegen ihrer guten Schäumeigenschaften und Reinigungswirkung für viele gewerbliche Anwendungen. Die geschmackliche Neutralität, die annehmbare Schleimhautverträglichkeit und die wachstumshemmende Wirkung auf Keime des Zahlbelages macht sie besonders geeignet als Tensidkomponente für die Herstellung von Mund-und Zahnpflegemitteln. Die Produkte werden für diese Zwecke als sprühgetrocknete oder umkristallisierte, feine Kristallpulver eingesetzt. Damit ist der Nachteil einer erheblichen Staubentwicklung beim Einsatz und eines unerwünschten Lufteintrages in das Produkt verbunden.

Einer Anwendung in gelöster Form steht die begrenzte Wasserlöslichkeit entgegen. Es ist schon vorgeschlagen worden, die Natrium-n-alkylsulfate mit Wasser und Glycerin und/oder Sorbit zu mischen, die erhaltene Suspension muß aber erst bei erhöhter Temperatur über einen längeren Zeitraum entgast werden, bevor eine Anwendung zur Zahnpastenherstellung möglich ist.

Es bestand daher ein Bedürfnis an einer wässrigen Zubereitung von Natrium-n-Alkylsulfaten in Form einer klaren Lösung mit niedrigem Trübungspunkt, die sich zur Anwendung der Natrium-n-Alkylsulfate - insbesondere zur Herstellung von Zahnpasten und Mundwässern-ohne die vorher genannten Nachteile eignet.

Es wurde gefunden, daß diese Aufgabe gelöst wird durch wässrige klarflüssige Zubereitungen von Natrium-n-Alkylsulfaten mit niedrigem Trübungspunkt, gekennzeichnet durch einen Gehalt von

5 - 20 Gew.-% Natrium-lauryl-und/oder - myristylsulfat

10 - 30 Gew.-% einer oberflächenaktiven, nichtionogenen Ethylenoxidanlagerungsverbindung mit einem HLB-Wert von 12 - 18

10 - 20 Gew.-% eines Polyols mit 3 - 6 C-Atomen und 2 - 6 Hydroxylgruppen oder eines Polyethylenglycols

30 - 75 Gew.-% Wasser

Neben dem obligatorischen Gehalt an Natriumlauryl und/oder Myristylsulfat sind untergeordnete Mengen an Natrium n-alkylsulfaten mit 8, 10, 16 oder 18 C-Atomen, wie sie als Nebenprodukt in technischen Natriumlaurylsulfaten auf Basis von Kokosfettalkoholschnitten enthalten sein können, durchaus nicht von Nachteil.

Als oberflächenaktive nichtionogene Ethylenoxidaddukte eignen sich Anlagerungsprodukte von Ethylenoxid an Fettsäuren, Fettsäure-Polyol-Partialester, z.B. an Mono und Diester von Glycerin, Pentaerythrit, und Sorbit der Fettsäuren mit 12 - 22 C-Atomen, der Ricinolsäure und der 9-Hydroxistearinsäure. Besonders gut eignen sich auch Oxethylierungsprodukte des Ricinusöls und des hydrierten Ricinusöls, also Umsetzungsprodukte von Triglyceriden mit Ethylenoxid.

Weiterhin eignen sich Anlagerunsprodukte von Ethylenoxid an Fettalkohole mit 12 - 22 C-Atomen, an Fettsäureamide oder Alkanolamide auf Basis von Fettsäuren mit 12 - 22 C-Atomen, und an Alkylphenole mit Alkylgruppen mit 8 - 12 C-Atomen.

Das Gewichtsverhältnis von hydrophilen zu lipophilen Gruppen in diesen Ethylenoxidaddukten sollte so sein, daß das Gewicht der (von Ethylenoxid und ggf. von Polyolen gebildeten) hydrophilen Glycolethergruppen etwa 60 - 80 Gew.-% des Gesamtmoleküls der Ethylenoxidaddukte ausmacht. Bei diesen, für die Herstellung erfindungsgemäßer Zubereitungen geeigneten Ethylenoxidaddukten liegt der HLB-Wert gemäß HLB = (E + P)/5 (wobei E = Gehalt Ethylenoxid in Gew.-% und P = Gehalt an Polyolether in Gew.-% im Addukt) im Bereich von 12 - 18.

Als Polyole mit 3 - 6 C-Atomen eignen sich z.B. 1.2-Propylenglycol, 1.3-Propylenglycol, Erythrit, Glycerin, Pentaerythrit, Neopentylglycol und Sorbit. Als Polyethylenglycole eignen sich bevorzugt flüssige Polyethylenglycole mit einem mittleren Molekulargewicht von 200 - 700. Im Hinblick auf den Einsatz in Zahnpasten sind Glycerin und Sorbit sowie Polyethylenglycole bevorzugt.

Besonders niedrige Trübungspunkte weisen erfindungsgemäße wässrige Zubereitungen auf mit einem Gehalt von

8 - 12 Gew.-% Natrium-lauryl-myristylsulfat auf Basis eines Lauryl-myristylalkoholgemisches aus 30 - 70 Gew.-% Lauryl und 30 - 70 Gew.-% Myritylalkohol

20 - 25 Gew.-% eines Ricinusöl-Oxethylats mit 60 - 70 Gew.-% Glycolethergruppen

12 - 18 Gew.-% Glycerin, Sorbit und/oder Polyethylenglycol

45 - 60 Gew.-% Wasser

auf.

Die erfindungsgemäßen klarflüssigen wässrigen Zubereitungen von Natrium-n-alkylsulfaten weisen Trübungspunkte unterhalb + 6 °C auf, so daß auch bei längerer Lagerung in kühlen Räumen keine Gefahr der Austrübung und Bodensatzbildung besteht. Sie eignen sich vorzüglich als Tensidkomponenten für die Herstellung von Mund-und Zahnpflegemitteln, da die Zugabe des Tensids in Form dieser Zubereitungen weder Staubbildung noch unerwünschter Eintrag von Luft in den Zahnpastenansatz verursacht. Die Komponen ten der erfindungsgemäßen Zubereitungen sind in Zahnpasten nicht störende, vielfach sogar erwünschte Bestandteile.

Die nachfolgenden Beispiele sollen den Gegenstand der Erfindung näher erläutern:

Beispiele

1. Die in der folgenden Tabelle aufgeführten, klaren wässrigen Zubereitungen wurden wie folgt hergestellt:

Eine Mischung aus Wasser und Polyol (oder Polyethylenglycol) wurde bei 20 °C vorgelegt, das Lauryl-myristylsulfat darin unter Rühren gelöst und schließlich das oberflächenaktive Ethylenoxidaddukt unter Rühren eingemischt.

Die Bestimmung des Kältetrübungspunktes der wässrigen Tensidzubereitungen erfolgte analog DIN ISO 3015.

Es wurden folgende Produkte mit Handelsbezeichnung benutzt:

Texapon [R]LS hochkonz.
Nadeln      : Natrium-lauryl-myristyl (C$_{12:14}$ = 70 : 30) sulfat

Eumulgin [R]RO 40      : Ricinusöl-oxethylat (ca. 40 Mol Ethylenoxid pro Mol Ricinusöl)

Eumulgin [R]SML 20      : Sorbitanmonolaurat-oxethylat (20 Mol Ethylenoxid)

Eumulgin [R]286      : Nonylphenoloxethylat (ca. 9,5 Mol Ethylenoxid)

Tabelle

| | | | | | | | | | | | | | | | |
|---|---|---|---|---|---|---|---|---|---|---|---|---|---|---|---|
| Texapon LS hochkonz. Nadeln | 10,2 | 10,2 | 9,0 | 20,0 | 20,0 | 10,2 | 17,0 | 9,0 | 10,2 | 17,0 | 10,2 | 17,0 | 10,2 | 10,2 | 17,0 |
| Eumulgin RO 40 | – | 25,0 | – | – | 25,0 | – | 23,3 | 25,0 | – | 25,0 | 23,3 | – | – | 25,0 | – |
| Eumulgin SML 20 | – | – | 23,3 | – | – | – | – | – | 15,0 | 23,3 | – | 15,0 | – | – | 23,3 |
| Eumulgin 286 | 23,3 | – | – | 23,3 | 23,3 | 23,3 | – | – | 23,3 | – | – | – | 15,0 | – | – |
| Glycerin | 15,0 | 15,0 | 15,0 | 15,0 | – | – | – | – | – | – | – | – | – | – | – |
| Sorbit (70%ig in Wasser) | – | – | – | – | 15,0 | 15,0 | 15,0 | – | – | – | – | – | – | – | – |
| Propylenglycol | – | – | – | – | – | – | – | 15,0 | 15,0 | 15,0 | – | – | – | – | – |
| Polyethylenglycol (mittl. Molgew. 200) | – | – | – | – | – | – | – | – | – | – | 15,0 | 15,0 | 15,0 | 15,0 | 15,0 |
| Wasser | 51,5 | 49,8 | 52,7 | 41,7 | 49,8 | 44,7 | 52,7 | 51,5 | 44,7 | 59,8 | 44,7 | 49,8 | 59,8 | 59,8 | 44,7 |
| Trübungspunkt liegt unterhalb | 0° | 0° | +6° | +6° | +6° | 0° | +6° | 0° | +6° | +6° | +6° | 0° | +6° | 0° | +6° |

**Ansprüche**

1. Wässrige klarflüssige Zubereitungen von Natrium-n-alkylsulfaten mit niedrigem Trübungspunkt, gekennzeichnet durch einen Gehalt von

5 - 20 Gew.-% Natrium-lauryl-und/oder myristylsulfat

10 - 30 Gew.-% einer oberflächenaktiven nichtionogenen Ethylenoxidanlagerungsverbindung mit einem HBL-Wert von 12 - 18

10 - 20 Gew.-% eines Polyols mit 3 - 6 C-Atomen und 2 - 6 Hydroxylgruppen oder eines Polyethylenglycols

30 - 75 Gew.-% Wasser

2. Zubereitung gemäß Anspruch 1, gekennzeichnet durch einen Gehalt von

8 - 12 Gew.-% Natrium-lauryl-myristylsulfat

20 - 25 Gew.-% eines Ricinusöl-Oxethylats mit 60 - 70 Gew.-% Glycolethergruppen

12 - 18 Gew.-% Glycerin, Sorbit oder Polyethylenglycol

45 - 60 Gew.-% Wasser

3. Verwendung der Zubereitungen gemäß Anspruch 1 und 2 als Tensidkomponente bei der Herstellung von Zahnpasten.